# EUROPÄISCHE PATENTSCHRIFT

(11) **EP 0 811 039 B1**
(45) Veröffentlichungstag und Bekanntmachung des Hinweises auf die Patenterteilung: **13.01.1999**
(21) Anmeldenummer: 96905775.1
(22) Anmeldetag: 21.02.1996
(51) Int. Cl.: C08L 83/04, C08L 71/00, C08L 81/04, A61K 6/10

(54) **ABFORMMASSE AUF SILIKONBASIS MIT WACHSZUSATZ**
IMPRESSION COMPOUND WITH A SILICON BASE AND WAX ADMIXTURE
MATIERE POUR EMPREINTES A BASE DE SILICONE AVEC ADDITION DE CIRE

(30) Priorität: 21.02.1995 DE 19505896; 16.05.1995 DE 19517962
(43) Veröffentlichungstag der Anmeldung: 10.12.1997
(73) Patentinhaber: ERNST MÜHLBAUER KG, D-22547 Hamburg (DE)
(72) Erfinder: LÜBBERS, Dierk, D-22523 Hamburg (DE); MÜHLBAUER, Wolfgang, D-22609 Hamburg (DE); HÜBNER, Heijo, D-82237 Wörthsee (DE)
(74) Vertreter: Glawe, Delfs, Moll & Partner
(86) Internationale Anmeldenummer: EP9600723
(87) Internationale Veröffentlichungsnummer: WO9626246

(56) Entgegenhaltungen:
- EP-A- 0 206 314
- EP-A- 0 212 144
- EP-A- 0 480 238
- US-A- 3 137 665
- US-A- 4 778 832
- US-A- 4 879 339
- US-A- 4 990 561
- DATABASE WPI Derwent Publications Ltd., London, GB; AN 90-112606 XP002007016 & JP,A,02 064 159 (DAINIPPON INK CHEM KK.) , 5.März 1990

## Beschreibung

Die Erfindung betrifft eine bei Raumtemperatur vulkanisierbare Masse auf der Basis von Polysiloxanen, Polysulfiden und/oder aziridinfreien Polyethern, deren Verwendung als Abformmasse sowie deren Herstellung.

In der Zahnmedizin wird anhand von Modellen Zahnersatz angefertigt. Ein solches Arbeitsmodell muß die Zahn- und Kieferverhältnisse originalgetreu wiedergeben. Zunächst wird mit Hilfe einer Abformmasse ein räumliches Negativ der Kiefersituation hergestellt. Die in einen plastischen Zustand überführte Abformmasse wird dazu mit einem Abdrucklöffel in den Mund des Patienten eingeführt und erstarrt dort zu einer elastischen Masse, die nach dem Herausnehmen die negative Form darstellt. Durch Ausgießen dieser Abformung mit einem Modellmaterial erhält man das Arbeitsmodell. Als Abformmassen werden bspw. Massen auf der Basis von Alginaten, Polysulfiden, Silikonen und vulkanisierbaren Polyethermaterialien verwendet.

Diese Massen haben verschiedene Eigenschaften und aufgrund ihrer Verarbeitbarkeit, ihrer physikalischen Eigenschaften, ihrer Qualität für die Abformung und Reproduktion und ihrer Kosten Vor- und Nachteile für jeden Anwendungsfall.

Solche bei Raumtemperatur vulkanisierbaren Abformmassen werden durch Vermischung eines Mittels hergestellt, bestehend aus ein oder mehreren Komponenten, meist und günstigerweise aus zwei, mindestens einer Basiskomponente und einer Härter- oder Initiator- oder Katalysatorkomponente. Basis und Härter sind Grundkomponenten des Mittels. Die nach der Vermischung eintretende gummielastische Verfestigung wird Vulkanisation, Polymerisation oder Abbindung genannt.

Weitverbreitet sind Abformmaterialien aus synthetischen Polymeren auf der Basis von Polysiloxanen (Silikonen). Sie werden durch eine chemische Reaktion zum Elastomer vernetzt. Man unterscheidet bei den Silikonmaterialien zwischen kondensationsund additionsvernetzenden Silikonmassen. Die kondensationsvernetzenden Silikone vernetzen in der Regel durch titan- oder zinnkatalysierte Reaktion des hydroxyterminierten Polysiloxans mit Siliciumalkoxyverbindungen unter Abspaltung eines Alkohols. Bei den additionsvernetzenden Silikonen erfolgt die Vernetzung durch in der Regel platinkatalysierte Reaktion von ungesättigten Kohlenwasserstoffendgruppen des Polysiloxans mit Si-H-Gruppen eines Hydrogenpolysiloxans (Hydrosilylierung). Solche Silikonabformmassen sind beispielsweise aus EP-A-0 162 211, DE-A-40 31 759 und EP-A- 0 166 107 bekannt. Die Silikonmassen werden in der Regel als Zweikomponentensystem geliefert, nach dem Vermischen der Komponenten wird die entstandene Masse in den Mund des Patienten eingebracht und härtet dort in einem Zeitraum von üblicherweise 3-5 Minuten aus.

Ein Nachteil der bekannten vulkanisierbaren Massen besteht darin, daß sie im noch nicht ausgehärteten Zustand eine verhältnismäßig geringe Standfestigkeit aufweisen. Diese läßt sich zwar durch Compoundieren mit geeigneten, chemisch verträglichen Füll- und Zusatzstoffen (bspw. Zusatz von pyrogenen Kieselsäuren oder einen hohen Befüllungsgrad der Paste mit groben anorganischen Füllstoffen) verbessern, jedoch ist damit auch meist ein thixotropes Verhalten oder eine mangelhafte Zeichnungsschärfe der Paste während der Abdrucknahme verbunden. Dies bedeutet, daß die Materialien unter der Wirkung des Anpressdruckes während der Abformung entweder dazu neigen, dem Druck auszuweichen und wegzufließen oder aber feine Details nicht abbilden können. Aufgrund dieser Eigenschaft ist auch das für die Wiedergabe der Details im Mund wichtige Eindringvermögen der Silikonabformmaterialien in Spalten nicht besonders gut.

Der Erfindung liegt die Aufgabe zugrunde, eine vulkanisierbare Masse der eingangs genannten Art zu schaffen, die eine verbesserte Standfestigkeit und Spalteneindringfähigkeit in noch nicht ausgehärtetem Zustand aufweist und daher die genannten Nachteile nicht oder in geringem Maße aufweist.

D.h., beim Anmischen (bspw. mittels eines Spatels auf einer Anmischunterlage) sollen die Komponenten der Abformmasse leicht fließen. Nach dem Anmischen soll aber die Masse möglichst eine Standfestigkeit erhalten, insbesondere soll die angemischte Masse nicht von der Anmischunterlage oder dem Applikationslöffel fließen, wenn dieser zwecks Abnahme eines Abdrucks im Unterkiefer auf den Kopf gestellt wird, gleichzeitig aber dem Druck nicht ausweichen, sondern vielmehr sich in die Details der Mundsituation einpressen. Bei herkömmlichen Abformmassen des Standes der Technik sind diese Eigenschaften jedoch nicht in dem wünschenswerten Maße vorhanden.

Erfindungsgemäß wird eine Masse auf Basis von Polysulfiden, Silikonen und/oder vulkanisierbarem aziridinfreien Polyethermaterial geschaffen, die durch einen Gehalt von wenigstens einem Wachs mit Ausnahme von hydriertem Rizinusöl, Paraffin- und Mikrowachsen gekennzeichnet ist. Die Masse ist erhältlich durch Erhitzen einer Mischung der Polysiloxane, Polysulfide und/oder aziridinfreien Polyethermaterialien sowie des Wachses auf eine Temperatur oberhalb des Schmelzpunktes des Wachses, Herstellen einer Emulsion durch Dispergieren und anschließendes Schockkühlen der Emulsion. Das Wachs bildet in der Masse ein kristallines Netzwerk.

Der Begriff Wachs soll im Rahmen der Erfindung alle Stoffe und Stoffgemische umfassen, die folgende Eigenschaften aufweisen:
- bei 20°C knetbar, fest bis brüchig hart,
- grob bis feinkristallin, durchscheinend bis opak, jedoch nicht glasartig,
- über 40°C ohne Zersetzung schmelzend,
- schon wenig oberhalb des Schmelzpunktes bzw. Erweichungspunktes verhältnismäßig niedrigviskos,
- stark temperaturabhängig in Konsistenz und Löslichkeit,
- unter leichtem Druck polierbar.

Diese Definition ist entnommen aus Ullmanns Enzyklopädie der technischen Chemie, 4. Auflage, Verlag Chemie, Weinheim, Band 24, Seite 3 und stellt eine in der Fachwelt anerkannte Definition von Wachsen dar. Ausgenommen aus dem Gegenstand der auf den Stoff sowie die unten näher beschriebenen Kits zu dessen Herstellung gerichteten Ansprüche sind Paraffin- und Mikrowachse, das heißt Wachse, die petrochemisch durch die Fraktionierung von Erdöl hergestellt werden. Paraffin- und Mikrowachse sind im einzelnen in der bereits genannten Ullmann Enzyklopädie, Band 24, Seite 22-24 beschrieben. Erfindungsgemäß können beispielsweise folgende Wachse verwendet werden.
- Pflanzenwachse, wie Carnauba-Wachse, Candelilla-Wachse, Ouricori-Wachse, Zuckerrohr-Wachse, Retamo-Wachse;
- tierische Wachse wie Bienenwachs, andere Insektenwachse oder Wollwachs;
- Montanwachse und Polyolefinwachse.

Eine Beschreibung geeigneter Wachse findet sich in dem genannten Band 24 von Ullmanns Enzyklopädie auf den Seiten 2-20 und 36-47. Diese Literaturstellen werden durch Bezugnahme darauf zum Gegenstand der Offenbarung dieser Anmeldung gemacht.

Wichtige Bestandteile der Wachse sind in der Regel eine oder mehrere der folgenden Komponenten:
Kohlenwasserstoffe, hauptsächlich gesättigte und wenig verzweigte Alkane; Alkylester; Alkylether; primäre Alkohole; Säuren; Ketone; Aldehyde; sekundäre Alkohole; Hydroxysäuren; Lactone; Acetale; Diole, hauptsächlich α-ω-Diole); Dicarbonsäuren; Diketone; Polyester.

Erfindungsgemäß können auch tierische oder pflanzliche Fette, ggf. hydrierte oder chemisch modifizierte Fette, die unter die oben angegebene Definition von Wachs fallen, verwendet werden. Eine Übersicht über Fette findet sich in Ullmanns Enzyklopädie der technischen Chemie, 4. Auflage, Band 11, Seite 457-524. Auch diese Literaturstelle wird durch Bezugnahme darauf zum Gegenstand der Offenbarung dieser Anmeldung gemacht. Als ein für die Zwecke der Erfindung besonders geeignetes Wachs hat sich ggf. hydrierter Rindertalg erwiesen.

Die erfindungsgemäßen Massen weisen ein gutes rheologisches Verhalten auf. Bei ihrer Verwendung als Abformmasse fließen sie an das abzuformende Modell unter geringem Druck dünnviskos an. Sie liegen an diesem Modell bis zu ihrer gummielastischen Erhärtung unverändert an und bleiben standfest, zwischenzeitliches Abfließen von der abzuformenden oralen Fläche findet nicht oder nur in geringem Maße statt.

Die Wirkung des erfindungsgemäß verwendeten Wachses besteht darin, daß der Wachs in der bei Raumtemperatur vulkanisierbaren Masse ein kristallines Netzwerk (mikrofeines Kristall- bzw. Nadelfilzgeflecht) bildet, das die Neigung der Masse zum Wegfließen unter Druck vermindert und so die Standfestigkeit erhöht. Aus EP-A-0 166 107 ist es zwar schon bekannt, Paraffin- oder Mikrowachse in Silikonabformmaterialien einzumischen. Aufgrund ihrer schlechten Kristallisationsneigung können diese Mikrowachse nicht durch Bildung eines kristallinen Netzwerks die Standfestigkeit der Masse verbessern, sie weisen nach EP-A-0 166 107 im Gegenteil einen so niedrigen Schmelzbereich auf, daß sie durch die Energiezufuhr beim Mischen und Kneten der Masse schmelzen oder zumindest weich werden und so die Klebrigkeit der Masse verhindern. Die zu lösende Aufgabe und die technische Wirkung der gemäß EP-A-0 166 107 zugesetzten Paraffin- oder Mikrowachse ist demnach eine andere als bei der vorliegenden Erfindung.

Aus EP-A 0 154 922 ist eine additionsvernetzende Masse auf Polysiloxanbasis bekannt, die hydriertes Rizinusöl als Strukturbildner enthält. Diese Druckschrift lehrt, das hydrierte Rizinusöl (Schmelzpunkt 85°C) unter Erwärmen auf 95°C in die Masse einzuarbeiten und anschließend unter Rühren die Masse auf 25°C abzukühlen. Von den Erfindern der vorliegenden Anmeldung durchgeführte Versuche haben gezeigt, daß nach dieser technischen Lehre ausschließlich hydriertes Rizinusöl, nicht jedoch andere Wachse, als Strukturbildner geeignet sind. Die vorliegende Erfindung hat erkannt, daß jegliche Wachse zur Erzielung der erfindungsgemäß gewünschten Wirkung geeignet sind, sofern die Herstellung der Massen nach dem weiter unten beschriebenen erfindungsgemäßen Verfahren erfolgt. Wesentlich für den Erfolg der Erfindung ist, daß nach dem Herstellen einer Lösung oder Emulsion von Wachs und bei Raumtemperatur vulkanisierbaren Masse durch Dispergieren ein verhältnismäßig rasches Abkühlen (Schockkühlen) der Emulsion bzw. Lösung erfolgt, vorzugsweise durch Aufbringen auf eine kalte Oberfläche. Überraschenderweise bewirkt dieses Schockkühlen, daß sich Massen mit dem erfindungsgemäß erwünschten Eigenschaften unter Zusatz von jeglichem Wachs herstellen lassen.

Der Wachsgehalt der Masse beträgt vorteilhafterweise 1-40 Gew.-%, vorzugsweise 2-15 Gew.-%, weiter vorzugsweise 5-10 Gew.-%. Die erfindungsgemäßen Wachse werden vorzugsweise in Polysiloxanen verwendet und können dort sowohl bei additionsvernetzenden als auch bei kondensationsvernetzenden Polysiloxanen eingesetzt werden. Die bei Raumtemperatur vulkanisierbare Masse findet als Abform,- Dublier- oder Modelliermasse insbesondere, jedoch nicht ausschließlich, im Dentalbereich Anwendung.

Der Schmelzpunkt bzw. Weichungsbereich der erfindungsgemäß verwendeten Wachse liegt bevorzugt zwischen 40 und 250°C, vorzugsweise beträgt er 40 bis 200°C, weiter vorzugsweise 40 bis 100°C, 40 bis 80°C, besonders bevorzugt sind 40 bis 70°C.

Das Molekulargewicht der verwendeten Wachse liegt vorzugsweise unter 2000. Des weiteren ist es bevorzugt, wenn die verwendeten Wachse Estergruppen enthalten. Beide genannten Merkmale tragen dazu bei, das Kristallisationsvermögen des Wachses oder der Wachse zu verbessern.

Der in den Ansprüchen verwendete Begriff "Masse auf der Basis von Polysiloxanen, Polysulfiden und/oder aziridinfreien Polyethern" soll alle Massen umfassen, bei denen nach der Vernetzung das polymere Netzwerk hauptsächlich Polysiloxane, Polysulfide und/oder aziridinfreie Polyether umfaßt. Der Begriff umschließt daher insbesondere auch solche Massen, bei denen beispielsweise der Gewichtsanteil von zugesetzten Füll- und Hilfsstoffen größer ist als der der Polysiloxane, Polysulfide und/oder aziridinfreien Polyether.

Dentale Abformmassen auf Silikonbasis werden üblicherweise als Kit in den Handel gebracht, das in der Regel zwei oder drei Komponenten aufweist. Nach Vermischen der Komponenten im vorgesehenen Verhältnis setzt die Vernetzung ein und läßt die Masse innerhalb weniger Minuten (üblicherweise 3-5 Minuten) zu einem elastischen Formkörper aushärten.

Gegenstand der Erfindung sind auch Kits zur Herstellung einer Masse gemäß einem der Ansprüche 1-6. Zum Zwecke der Herstellung einer additionsvernetzenden Polysiloxanmasse weisen zwei Komponenten des Kits erfindungsgemäß jeweils die folgenden Bestandteile auf:
Komponente 1:
   - Polysiloxane mit mindestens zwei ungesättigten Kohlenwasserstoffgruppen, vorzugsweise Vinylgruppen, im Molekül,
   - Hydrogenpolysiloxane mit mindestens zwei Si-H-Gruppen im Molekül,
   - Wachse mit Ausnahme von hydriertem Rizinusöl, Paraffinoder Mikrowachsen,
   - gegebenenfalls übliche Füllstoffe sowie Zusatz-, Hilfsund Farbstoffe,
Komponente 2:
   - gegebenenfalls Polysiloxane mit mindestens zwei ungesättigten Kohlenwasserstoffgruppen, vorzugsweise Vinylgruppen, im Molekül,
   - Katalysator,
   - gegebenenfalls Wachse mit Ausnahme von hydriertem Rizinusöl, Paraffin- oder Mikrowachsen,
   - gegebenenfalls übliche Füllstoffe sowie Zusatz-, Hilfsund Farbstoffe,
   wobei die Wachse in jeder wachshaltigen Komponente ein kristallines Netzwerk bilden.

Komponente 1 enthält die beiden miteinander vernetzbaren Polysiloxankomponenten, nämlich die mit reaktiven Doppelbindungen (in der Regel Vinylendgruppen) versehenen Polysiloxane, vorzugsweise sogenannte vinylendgestoppte Polysiloxane und die mit reaktiven Si-H-Gruppen versehenen Hydrogenpolysiloxane. Ferner enthält Komponente 1 den erfindungsgemäßen Wachszusatz sowie in der Regel übliche Füllstoffe wie beispielsweise Quarz- und β-Cristobalitmehle, Calciumsulfat und -carbonat, Diatomeenerde und pyrogene Kieselsäure. Der Anteil dieser Füllstoffe liegt üblicherweise zwischen 5 und 60 Gew.-%.

Die Zusatz- und Hilfsstoffe können beispielsweise übliche Weichmacher, Paraffinöle oder ähnliches sein, ferner kann es sich auch um Polysiloxane ohne reaktive Gruppen handeln, die also an der Vernetzungsreaktion nicht teilnehmen können. Es können zu diesem Zweck beispielsweise Trimethylsilyl-endge-stoppte Polysiloxane verwendet werden.

Farbstoffe werden insbesondere zur Unterscheidung der beiden Komponenten (Basis- und Katalysatorpaste) und zur Mischkontrolle eingesetzt. In der Regel werden anorganische oder organische Farbpigmente verwendet.

Die in Komponente 2 ggf. enthaltenen Polysiloxane sowie die gegebenenfalls vorhandenen Wachse, Füllstoffe und sonstige Zusätze entsprechen den gerade beschriebenen Bestandteilen.

Bei dem Katalysator handelt es sich bevorzugt um einen Platinkomplex, der beispielsweise aus Hexachloroplatin(IV)-Säure hergestellt werden kann. Jedoch ist auch jede andere Platinoder eine andere Katalysatorverbindung geeignet, die die Additionsvernetzungsreaktion beschleunigt. Besonders gut geeignet sind Platin-Siloxan-Komplexe, die beispielsweise in US-A-3 715 334, US-A-3 775 352 und US-A-3 814 730 beschrieben werden.

Insbesondere wenn eine längere Lagerfähigkeit der Komponenten gewünscht ist, sollte das in Komponente 2 gegebenenfalls eingearbeitete Wachs in Hinblick auf die chemische Verträglichkeit mit dem verwendeten Katalysator ausgewählt werden, damit dieser nicht vorzeitig unwirksam wird. Als Zusatzstoffe können gegebenenfalls auch übliche Katalysatorinhibitoren zugesetzt werden, um die Lagerfähigkeit der Katalysatorpaste zu erhöhen.

Die in den Ansprüchen verwendete Formulierung, daß das Kit zwei Komponenten enthält, bedeutet keine abschließende Aufzählung der Komponenten eines erfindungsgemäßen Kits. Zusätzliche Komponenten können ggf. vorhanden sein.

Vorteilhafterweise ist das additionsvernetzende Kit so abgestimmt, daß Basis- und Katalysatorpaste in einem Verhältnis von 1:1 miteinander gemischt werden. Dies stellt eine wesentliche Erleichterung beim Anmischen der Masse dar, da dann lediglich gleichlange Stränge der beiden Pasten miteinander vermischt werden müssen. Vorteilhafterweise liegt dann der Wachsgehalt in jeder der beiden Komponenten zwischen 1 und 40 Gew.-%, vorzugsweise zwischen 2 und 15 Gew.-%, weiter vorzugsweise zwischen 5 und 10 Gew.-%. Auch der Füllstoffanteil liegt dann in beiden Komponenten vorteilhafterweise zwischen den bereits genannten Werten von 5 und 60 Gew.-%.

Es sei angemerkt, daß die Mengenverhältnisse der beiden Komponenten variieren können. So ist es beispielsweise nicht unbedingt erforderlich, daß auch in der Katalysatorpaste Wachse enthalten sind. Wenn beispielsweise die Einstellung der Pasten so erfolgt, daß nur geringe Mengen der Katalysatorpaste mit der Basispaste vermischt werden müssen, ist ein Wachszusatz in der Katalysatorpaste entbehrlich. Entscheidend bei diesem Kit ist lediglich, daß nicht von vornherein beide miteinander vernetzenden Polysiloxankomponenten und der Katalysator in einer Komponente enthalten sein dürfen. Im Rahmen der Erfindung kann somit die Komponente 2 auch ausschließlich einen Katalysator enthalten.

Bei einem erfindungsgemäßen Kit zur Herstellung einer kondensationsvernetzenden Silikonmasse, weisen zwei Komponenten die jeweils folgenden Bestandteile auf:
Komponente 1:
   - Polysiloxane mit mindestens zwei Hydroxygruppen im Molekül,
   - Wachse mit Ausnahme von Paraffin- oder Mikrowachsen,
   - gegebenenfalls übliche Füllstoffe sowie Zusatz-, Hilfsund Farbstoffe,
Komponente 2:
   - Polyalkoxysilicate mit mindestens zwei Alkoxygruppen im Molekül,
   - Katalysator,
   - gegebenenfalls Wachse mit Ausnahme von Paraffin- oder Mikrowachsen,
   - gegebenenfalls übliche Füllstoffe sowie Zusatz-, Hilfsund Farbstoffe,
   wobei die Wachse in jeder wachshaltigen Komponente ein kristallines Netzwerk bilden.

Die Wachse und gegebenenfalls die beigefügten Füll- und Zusatzstoffe sind die gleichen wie vorstehend im Zusammenhang mit den additionsvernetzenden Silikonen beschrieben. Bei den in Komponente 2 enthaltenen Polyalkoxysilikaten handelt es sich um vernetzend wirkende Kieselsäureester (in der Regel Ethylester), die unter der Wirkung des Katalysators unter Alkoholabspaltung mit den Hydroxygruppen des in Komponente 1 enthaltenen Polysiloxans kondensieren können.

Als Katalysator für die Kondensationsreaktion werden beispielsweise organische Zinn- oder Titanverbindungen wie Zinnoctoat oder Dibutylzinn-Dilaurat verwendet.

Bei den kondensationsvernetzenden Kits wird in der Regel die Katalysatorpaste (Komponente 2) der Basispaste (Komponente 1) in einem deutlich geringeren Anteil als 1:1 zugemischt. Üblich ist beispielsweise ein Mischungsverhältnis von 1:10. Es wird daher häufig nicht erforderlich sein, auch der Komponente 2 Wachse und sonstige Füll- und Zusatzstoffe beizufügen. Im Rahmen der Erfindung ist es daher auch möglich, als Komponente 2 bei den kondensationsvernetzenden Systemen eine handelsübliche Katalysatorpaste einzusetzen. Weiterhin ist es im Rahmen der Erfindung möglich, daß die Polysiloxane mit mindestens zwei Hydroxygruppen im Molekül einerseits und die Polyalkoxysilicate andererseits Bestandteile einer Komponente sind und daß der Katalysator Bestandteil einer zweiten Komponente ist.

Vorteilhafterweise ist der Wachsgehalt der Komponenten 1 und/oder 2 so, daß sich nach deren Vermischen im vorgesehenen Verhältnis in der erhaltenen Masse ein Wachsgehalt von 1-40 Gew.-%, vorzugsweise 2-5 Gew.-%, weiter vorzugsweise 5-10 Gew.-% einstellt.

Bei den beschriebenen Kits wird Lagerstabilität u.a. dadurch erreicht, daß die an der Polymerisationsreaktion teilnehmenden bzw. diese Reaktion initiierenden Basis- und Katalysatorkomponenten physikalisch voneinander getrennt gelagert werden. Alternativ ist es denkbar, diese Komponenten nicht physikalisch voneinander zu trennen, sondern unter Bedingungen zu lagern, die eine Polymerisation ausschließen, wie bspw. Luft-, Feuchtigkeits- und/oder Lichtausschluß. Lagerstabilität bedeutet ferner, daß die Bestandteile der Komponenten des Kits unter üblichen Umgebungsbedingungen (Atmosphärendruck und Temperaturen bis 30°C) bis zur Bereitstellung für die bestimmungsgemäße Vermischung stabil gegenüber Endmischungen sind und keine chemischen Reaktionen miteinander eingehen.

Ein weiterer Gegenstand der Erfindung ist ein Verfahren zur Herstellung der wachshaltigen Komponenten eines zur Herstellung aziridinfreien, bei Raumtemperatur vulkanisierbare Masse vorgesehenen Kits. Es weist die folgenden Verfahrensschritte auf:
- Erhitzen einer Mischung von Polysiloxanen, Polysulfiden und/oder aziridinfreien Polyethermaterialien sowie von Wachs, mit Ausnahme von Paraffin- oder Mikrowachsen, sowie gegebenenfalls von Füll-, Zusatz-, Hilfsund Farbstoffen auf eine Temperatur oberhalb des Schmelzpunktes des Wachses oder der Wachse,
- Herstellen einer Emulsion von Wachs mit Ausnahme von Paraffin- oder Mikrowachsen, und Polysiloxane, Polysulfiden und/oder aziridinfreien Polyethermaterialien durch Dispergieren,
- Schockkühlen der Emulsion,
- gegebenenfalls Einmischen weiterer Füll-, Zusatz-, Hilfs- und Farbstoffe in die abgekühlte Masse.

Entscheidend für den Erfolg des erfindungsgemäßen Verfahrens ist zunächst das vorgesehene Erhitzen der Mischung auf eine Temperatur oberhalb des Schmelzpunktes des Wachses. Wenn das verwendete Wachs keinen scharfen Schmelzpunkt, sondern einen breiteren Schmelzbereich aufweist, ist dieses Merkmal so zu verstehen, daß die Mischung auf eine Temperatur oberhalb dieses Schmelzbereichs erwärmt wird. Das Wachs muß insgesamt in die flüssige Phase übergegangen sein. Ein vorher in die festen Phase des Wachses vorhandenes kristallines Netzwerk muß zumindest weitgehend zerstört sein.

Da sich Wachs einerseits und Polysiloxane, Polysulfide und/oder aziridinfreie Polyethermaterialien andererseits in der Regel nicht ineinander lösen, liegt jetzt eine Zwei-Phasen-Mischung vor, die durch Dispergieren in eine Emulsion überführt werden muß. Sofern sich die Bestandteile der Mischung ineinander lösen, ist der Dispergierschritt entbehrlich. Sofern die Mischung schon sonstige Füll- oder Zusatzstoffe aufweist, sind diese entweder in einer der beiden Phasen gelöst oder in der Mischung dispergiert. In der Regel ist es jedoch vorteilhaft, insbesondere anorganische Füllstoffe erst im letzten obengenannten Verfahrensschritt zuzufügen. Die Bildung und Stabilisierung der Emulsion kann gegebenenfalls durch bekannte und übliche oberflächenaktive Stoffe wie Emulgatoren, Emulsionsstabilisatoren oder pyrogene Kieselsäuren unterstützt werden.

Im nächsten Verfahrensschritt wird die hergestellte Emulsion bzw. Lösung schockgekühlt. Bei der Abkühlung wird der Schmelzpunkt bzw. Schmelzbereich des Wachses unterschritten. Das Abkühlen erfolgt schnell (Schockkühlen), beispielsweise durch Aufbringen der Emulsion auf eine kalte Oberfläche wie eine Kühlwalze. Beim Schockkühlen bildet das in der Mischung enthaltene Wachs ein kristallines Netzwerk, das die Masse insgesamt durchdringt und so zu der erfindungsgemäß verbesserten Standfestigkeit der Masse führt. Besonders vorteilhaft ist es, wenn vor dem Schockkühlen eine klare Lösung oder feststofffreie Emulsion vorliegt und das Wachs erst beim Schockkühlen auskristallisiert und das kristalline Netzwerk bildet.

Ein vorteilhaftes kontinuierliches technisches Verfahren zum Schockkühlen ist das Aufbringen der heißen Lösung bzw. Emulsion auf einen gut kühlbaren Ein- bis Dreiwalzenstuhl ggf. unter Schutzgas oder unter Luftausschluß. Die Zulaufmenge von Lösung bzw. Emulsion und die Betriebsbedingungen des Walzenstuhls werden vorzugsweise so gewählt, daß die entnommene schockgekühlte Vormischung eine Temperatur nicht wesentlich oberhalb der Raumtemperatur aufweist. Die Abnahmetemperatur läßt sich vorteilhafterweise durch die Dicke der schockzukühlenden aufgetragenen Schicht und die Veränderung der Umlaufgeschwindigkeit der Kühlwalzen regeln.

In die abgekühlte Masse können mit Hilfe üblicher Compoundierungsmethoden gegebenenfalls weitere übliche Füll-, Hilfs-, Zusatz- und Farbstoffe eingebracht werden. Häufig ist es vorteilhaft, wenn man vor dem Einarbeiten der Zusatzstoffe die Vormischung bei einer Temperatur von unter 20°C 24 Stunden ruhen und reifen läßt.

Die gemäß dem erfindungsgemäßen Verfahren hergestellten Massen zeigen ein gutes rheologisches Verhalten. Die Komponenten eines Kits lassen sich beim Anmischen mühelos homogen vermischen, die Zähigkeit der Komponenten des Kits ist gering. Die nach dem Anmischen der Komponenten erhaltene homogenisierte und noch nicht ausgehärtete Abformmasse ist nicht oder nur geringfügig fadenziehend und weist eine ausreichende Standfestigkeit auf, so daß sie nicht vom Abformlöffel fließt. Die noch nicht polymerisierte Masse läßt sich mittels einer feindüsigen Spritze in abzuformende Hohlräume und Hinterschnitte des abzuformenden Modells einbringen.

Die mit den erfindungsgemäßen Abformmaterialien hergestellten Abformungen weisen eine gute Zeichnungsschärfe auf. Bei der Verwendung im Oralbereich weisen die erfindungsgemäßen Abformmassen aufgrund ihrer relativen Hydrophobie eine gute Entformbarkeit auf.

Die wachshaltigen Komponenten zur Herstellung erfindungsgemäßer Abformmassen zeigen als dünne Schicht im Durchlichtmikroskop unter polarisiertem Licht eine fein polykristalline Struktur. Diese polykristalline Struktur verschwindet beim Erwärmen. Wenn anschließend ein langsames Abkühlen erfolgt, zeigt das Material eine sehr viel gröber kristalline Struktur. Der Vorgang ist reversibel, beim anschließenden Erwärmen und Schockkühlen entsteht wieder die ursprüngliche feinkristalline Struktur. Somit eignet sich die Beobachtung mittels Durchlichtmikroskop auch zur Qualitätskontrolle und Überwachung einer Produktion.

Die Erfindung wird nachfolgend anhand von Ausführungsbeispielen erläutert. In den beigefügten Figuren zeigen:
- Fig. 1: schematisch eine Vorrichtung zum Prüfen der Standfestigkeit einer Silikonmasse;
- Fig. 2: das Ergebnis der Prüfung der Standfestigkeit einer kondensationsvernetzenden Silikonmasse;
- Fig. 3: das Ergebnis der Prüfung der Standfestigkeit der Basispaste einer additionsvernetzenden Silikonmasse.

### Beispiel 1

Basis- und Katalysatorpaste für eine erfindungsgemäße additionsvernetzende Silikonmasse werden wie folgt hergestellt:

### Basispaste:

113 g vinylendgestopptes Polydimethylsiloxan mit einer Viskosität von 1000 mPas, 57 g vinylendgestopptes Polydimethylsiloxan mit einer Viskosität von 65000 mPas, 14 g vinylendgestopptes Polydimethylsiloxan mit einer Viskosität von 165000 mPas (Silopren U1, U65 bzw. U165, Bayer AG, Leverkusen), 19 g Paraffin (Paraffin, dünnflüssig, Pioneer 2076, Fa. Hansen & Rosenthal, Hamburg), 5 g ethoxylierter Fettalkohol (Lutensol A3, BASF AG), 22 g pyrogene Kieselsäure HDKH 2000/4 (Wacker AG), 13 g Polyhydrogensiloxan mit einem Gehalt an SiH-Gruppen von 4,3 mmol/g und 25 g hydrierter Rindertalg Ewanol HY 2 (Handelsname der Firma E.Wagner, Bremen) werden in ein 500 ml Becherglas eingewogen und im Wärmeschrank 1 h bei 150°C erhitzt.

Dann wird der Ansatz im heißen Zustand 2 min mit einem Dispergierer homogen gerührt und erneut 30 Minuten lang im Wärmeschrank bei 150°C erhitzt. Im heißen Zustand wird die erhaltene Masse über einen Dreiwalzenstuhl (EXAKT 50, Exakt Apparatebau, Norderstedt, Walzendurchmesser 50 mm, Walzenlänge 15 cm) gegeben. Die Walzen wurden bei Raumtemperatur und niedrigster Drehgeschwindigkeit betrieben. Dann läßt man die erhaltene Harzmischung über Nacht bei Raumtemperatur nachreifen. Am nächsten Tag wird mit einem Planetenkneter eine Füllstoff mischung aus 15 g Kieselgur, 63 g β-Cristobalit Sikron SF 6000 (Quarzwerke Frechen) und 146 g Sikron SF 8000 in die Harz mischung eingearbeitet.

Die Paste zeigt eine besondere Standfestigkeit, die sich zum Beispiel dadurch bemerkbar macht, daß auf dem Mischblock geformte Strukturen auch nach einigen Tagen nicht zusammengeflossen sind.

### Katalysatorpaste:

Analog dem Vorgehen zur Herstellung der Basispaste werden 117, 96 und 11 g vinylendgestoppte Polydimethylsiloxane der Viskosität 1000, 65000 und 165000 mPas, 20 g Paraffin (Paraffin, dünnflüssig, Pionier 2076, Fa. Hansen & Rosenthal, Hamburg), 5 g ethoxylierter Fettalkohol (Lutensol A3, BASF AG), und 23 g pyrogene Kieselsäure HDKH 2000/4 mit 25 g Ewanol HY2 (Handelsname der Firma E.Wagner, Bremen) aufgeschmolzen, dispergiert und über einen Dreiwalzenstuhl gegeben. Am nächsten Tag werden 15 g Kieselgur (PF5, Fa. Meyer-Breloh, Münster), 186 g Sikron SF 6000, 20 g Sikron SF 8000, 0,5 g Titandioxid (Kronos AV, Fa. Kronos, Leverkusen), 0,2 g Divinyltetramethyldisiloxan sowie 6,5 g eines Komplexes aus Platin und Divinyltetramethyl disiloxan mit einem Planetenkneter in die Harzmischung eingearbeitet. Die so erhaltene Katalysatorpaste zeigt dieselbe Standfestigkeit wie die Basispaste in Beispiel 1.

Mischt man diese Katalysatorpaste im Gewichtsverhältnis 1:1 mit der Basispaste, so härtet die Mischung nach wenigen Minuten zu einem elastischen, reißfesten Formkörper mit einer Shore A Härte (nach 24h) von ca 50 aus. Das Material hat einen linearen Schrumpf von weniger als 0,1% (bestimmt nach ISO 4823).

### Vergleichsbeispiel 1 (nicht erfindungsgemäß)

### Basispaste:

113 g vinylendgestopptes Polydimethylsiloxan mit einer Viskosität von 1000 mPas, 57 g vinylendgestopptes Polydimethylsiloxan mit einer Viskosität von 65000 mPas, 14 g vinylendgestopptes Polydimethylsiloxan mit einer Viskosität von 165000 mPas, 19 g Paraffin, 5 g ethoxylierter Fettalkohol (Lutensol A3, BASF AG), 22 g pyrogene Kieselsäure HDKH 2000/4 und 13 g Polyhydrogensiloxan mit einem Gehalt an SiH - Gruppen von 4,3 mmol/g werden auf einem Planetenkneter mit einer Füllstoffmischung aus 15 g Kieselgur, 63 g β-Cristobalit Sikron SF 6000 (Quarzwerke Frechen) und 146 g Sikron SF 8000 zu einer homogenen Paste verarbeitet.

### Katalysatorpaste:

Analog dem Vorgehen zur Herstellung der Basispaste werden 117, 96 und 11 g vinylendgestoppte Polydimethylsiloxane der Viskosität 1000, 65000 und 165000 mPas, 20 g Paraffin, 5 g ethoxy lierter Fettalkohol (Lutensol A3, BASF AG), und 23 g pyrogene Kieselsäure HDKH 2000/4 werden mit 15 g Kieselgur, 186 g Sikron SF 6000, 20 g Sikron SF 8000, 0,5 g Titandioxid, 0,2 g Divinyltetramethyldisiloxan und 6,5 g eines Komplexes aus Platin und Divinyltetramethyldisiloxan mit einem Planetenkneter zu einer Paste verarbeitet.

Mischt man diese Katalysatorpaste im Gewichtsverhältnis 1:1 mit der Basispaste aus Beispiel 1, so härtet die Mischung nach wenigen Minuten zu einem elastischen, reißfesten Formkörper mit einer Shore A Härte (nach 24h) von ca. 50 aus. Das Material hat einen linearen Schrumpf von weniger als 0,1% (bestimmt nach ISO 4823).

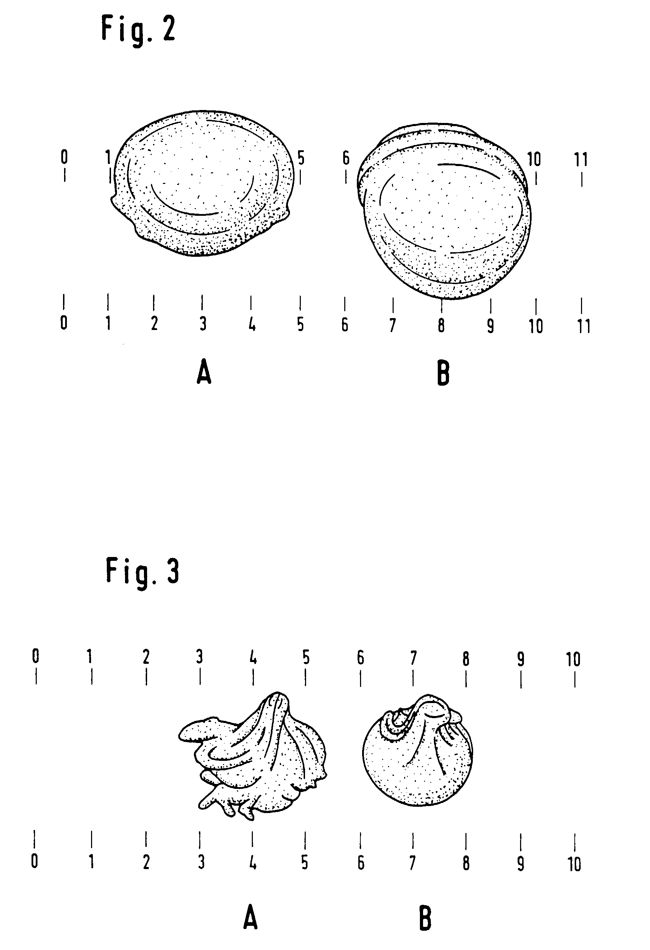

Fig. 3 zeigt die Standfestigkeit der nach Beispiel 1 (A) bzw. Vergleichsbeispiel (B) hergestellten Basispaste. Es sei angemerkt, daß vergleichbare Ergebnisse auch für die Katalysatorpaste erhalten werden. In beide Basispasten wurden auf dem Mischblock mit Hilfe eines Spatels Strukturen eingeformt. Fig. 3 zeigt, daß diese Strukturen bei der erfindungsgemäßen Basispaste nach einer Stunde im wesentlichen noch unverändert vorhanden sind, während sie bei der Basispaste des Standes der Technik großenteils bereits wieder zusammengeflossen sind.

### Beispiel 2

Dieses Beispiel zeigt ein erfindungsgemäßes Zweikomponentensystem zur Herstellung einer kondensationsvernetzenden Silikonmasse.

### Basispaste:

115 g hydroxylterminiertes Polydimethylsiloxan mit einer Viskosität von 2000 mPas, 119 g hydroxylterminiertes Polydimethylsiloxan mit einer Viskosität von 18000 mPas (Silopren C2 bzw. C18, Bayer AG, Leverkusen), 4 g pyrogene Kieselsäure HDKH 2000/4 (Handelsprodukt der Firma Wacker) und 19 g hydrierter Rindertalg Ewanol HY 2 (Handelsname der Firma E. Wagner, Bremen) werden in ein 500 ml Becherglas eingewogen und im Wärmeschrank 1 h bei 140°C erhitzt.
Dann wird der Ansatz im heißen Zustand 2 min mit einem Dispergierer homogen gerührt und erneut 30 Minuten lang im Wärmeschrank bei 140°C erhitzt. Im heißen Zustand wird die erhaltene Masse über einen Dreiwalzenstuhl gegeben. Dann läßt man die erhaltene Harzmischung über Nacht bei Raumtemperatur nachreifen. Am nächsten Tag wird mit einem Planetenkneter eine Füllstoffmischung aus 31 g β-Cristobalit Sikron SF 8000, 121 g Silbond 8000 RST (Quarzwerke Frechen), und 3,6 g Farbpigment in die Harzmischung eingearbeitet. Zusätzlich werden 1 g destilliertes Wasser, 5 g Glycerin und 2 g Silikonöl Silopren AC 3031 (Handelsprodukt der Bayer AG) in die Paste eingerührt.

Die Paste zeigt etwa die gleiche Standfestigkeit, wie die in Fig. 3 unter A gezeigte erfindungsgemäße additionsvernetzende Basispaste.

Als Katalysatorkomponente wurde der im Handel erhältliche Pastenhärter "Silagum KV Katalysator" (Fa. DMG, Hamburg) verwendet und im Gewichtsverhältnis 1:10 mit der Basispaste angemischt. Dieser Katalysator ist eine Mischung aus alkoxylierten Polysilikaten, Tetraethylorthosilicat, Zinnkatalysatoren, Vaseline, dünnflüssigem Paraffin und pyrogener Kieselsäure. Die Mischung härtet nach wenigen Minuten zu einem elastischen, reißfesten Formkörper mit einer Shore A Härte (nach 24 h) von ca. 40 aus.

### Vergleichsbeispiel 2 (nicht erfindungsgemäß)

### Basispaste:

Die Komponenten aus Beispiel 2 wurden mit Ausnahme des hydrierten Rindertalgs Ewanol HY 2 und ohne den beschriebenen Schmelz- und Abkühlvorgang im Planetenkneter zu einer Paste gemischt. Die erhaltene Paste zeigt eine deutlich schlechtere Standfestigkeit als die Paste aus Beispiel 2. Auf dem Mischblock geformte Strukturen zerfließen nach einigen Stunden wieder.

Als Katalysatorkomponente wurde der im Handel erhältliche Pastenhärter "Silagum KV Katalysator" verwendet und im Gewichtsverhältnis 1:10 mit der Basispaste angemischt. Die Mischung härtet nach wenigen Minuten zu einem elastischen, reißfesten Formkörper mit einer Shore A Härte (nach 24 h) von ca. 40 aus.

### Vergleichsbeispiel 3 (nicht erfindungsgemäß)

Zwei Organopolysiloxan-Gemische, "Gemisch A" und "Gemisch B", werden gemäß DE-OS 27 36 421 Beispiel 1 hergestellt.

### Gemisch A (Katalysatorpaste)

149 Teile eines Organopolysiloxans aus 71,6 Molprozent Einheiten der Formel CH₃SiO_{3/2}, 20,6 Molprozent Einheiten der Formel (CH₃)₂SiO und 7,8 Molprozent Einheiten der Formel CH₂=CH(CH₃)₂SiO_{1/2} mit einer Viskosität von 97000 cP bei 25°C werden mit 2,62 Teilen der 1 Gew.-% Platin enthaltenden Mischung aus Platin-Divinyltetramethyldisiloxan-Komplex und Dimethylpolysiloxan mit einer Viskosität von 1400 cP bei 25°C, deren Herstellung oben beschrieben wurde, 1,64 Teilen einer 2 gew.-%igen Lösung von 1,3-Divinyl-1,1,3,3-tetramethyldisiloxan in dem vorstehend genannten Organopolysiloxan mit Monomethylsiloxaneinheiten, 149 Teilen Quarzmehl und 25 Teilen eines handelsüblichen, pyrogen in der Gasphase erzeugten Siliciumdioxids mit einer Oberfläche von 200 m²/g, die zu 60 % durch Behandlung mit Dimethyldichlorsilan hydrophobiert ist, vermischt.

### Gemisch B (Basispaste)

121 Teile des Organopolysiloxans mit Monomethylsiloxaneinheiten, das im Zusammenhang mit der Beschreibung der Herstellung des Gemischs A näher definiert wurde, werden mit 50 Teilen eines durch Trimethylsiloxygruppen endblockierten Organopolysiloxans aus 66,6 Molprozent Methylhydrogensiloxaneinheiten und 33,4 Molprozent Dimethylsiloxaneinheiten mit einer Viskosität von 80 cP bei 25°C, 121 Teilen Quarzmehl und 35 Teilen des im Zusammenhang mit der Beschreibung der Herstellung des Gemischs A näher definierten, teilweise hydrophobierten Siliciumdioxids vermischt.

Sowohl Gemisch A als auch Gemisch B werden einzeln auf einer planen Unterlage oder einem Anmischblock ausgestrichen. Beide Pasten sind zäh und weichen dem Druck des Anmischspatels nur wiederstrebend aus. Die Pasten sind fadenziehend.

Bei einer Vermischung 1:1 von A und B lassen sich die Pasten auch nur widerstrebend mit dem Spatel vermischen. Die Zeichnungsschärfe an oralen Flächen, besonders bei Übergängen von Zahnhälsen zur Schleimhaut und sogenannten Taschen, ist gering.

Sowohl Gemisch A als auch Gemisch B lassen sich nur mit großen Mühen aus einer Applikationsspritze ausbringen. Dasselbe gilt für das vulkanisierende Gemisch unmittelbar nach dem Vermischen.

### Beispiel 3

### Herstellung der Kristallfilz-Pasten:

Wie näher in vorgenannter DE-OS 27 36 421, Beispiel 1, definiert, werden jeweils ein Organopolysiloxan gemäß Gemisch A ohne Platinkatalysator und gemäß Gemisch B zu gleichen Teilen vermischt und mit trimethylsilylendgestoppten Polydimethylsiloxan der Viskosität 50 cP (25°C) auf eine Viskosität von 25000 mPa*s eingestellt. Anschließend werden 171 Teile davon und 5 Teile pyrogener Kieselsäure, 17,1 Teile hydrierter Rindertalg mit einem Erweichungspunkt von 48 - 52°C in ein Behältnis eingewogen und unter intensivem Rühren auf 60°C erwärmt, bis eine homogene Emulsion entsteht.

Die so erhaltene Emulsion wird sodann in einem dünnen Strahl auf einen nicht zu schnell drehenden Drei-Walzenstuhl gegeben, dessen Walzen von innen gekühlt werden. Es kann dann an der dritten Walze über einen Abschaber eine farblos opake, steife Paste entnommen werden, die 18 bis 20°C hat.

Die so erhaltene Vormischung läßt man einen Tag an einem kühlen Ort ruhen.

### Erfindungsgemäßes Gemisch:

188 Teile der Vormischung werden dann in einem Kneter mit nicht zu hohen Scherkräften mit 19 Teilen Quarzmehl versetzt, anschließend die erhaltene Paste weiter homogenisiert und endlich durch eine Vakuum-Knetung blasenfrei gemacht. Die Herstellung erfolgt unter Stickstoff als Schutzgas.

Das so erhaltene Gemisch läßt sich auf einer planen Unterlage oder einem Anmischblock sehr leicht ausstreichen. Die Paste läßt sich leicht bewegen und weicht dem Druck des Anmischspatels hervorragend aus. Andererseits hat sie eine hervorragende Standfestigkeit.

Die Paste reißt sofort vom Spatel ab, wenn dieser aus der Masse gezogen wird. Sie ist deshalb nicht fadenziehend und eine blasenfreie Vermischung mit dem Katalysator ist in hervorragender Weise gewährleistet.

Eine Vermischung von 200 Teilen des Gemisches mit 2 Teilen des Platin-Katalysators nach Beispiel 1 aus DE-OS 27 36 421 kann schnell und leicht erfolgen. Die Zeichnungsschärfe an oralen Flächen, besonders bei Übergängen von Zahnhälsen zur Schleimhaut und sogenannten Taschen, ist hervorragend.

Die Zeichnungsschärfe ist dann besonders hoch, wenn das angemischte Material vor der Vulkanisation mit einer Applikationsspritze in die Nischen und Taschen des abzuformenden Modells verbracht wird und das so vorbereitete Modell erst anschließend in die Abformmasse getaucht wird.

Das Gemisch läßt sich leicht aus einer Applikationsspritze ausbringen. Dasselbe gilt für das vulkanisierende Gemisch unmittelbar nach dem Vermischen.

### Vergleichsbeispiel und Beispiel 4

Wie im sogenannten erfindungsgemäßen Beispiel, Herstellung des Harzes, der DE-OS 44 39 769 näher definiert, wird ein vulkanisierbares Harz hergestellt.

### Vergleichsbeispiel 4

770 Teile des in DE-OS 44 39 769 beschriebenen Harzes werden mit 210 Teilen Kieselgur Celatom MW 25 und 20 Teilen pyrogener Kieselsäure Cabosil TS 610 zu einer Paste verknetet, homogenisiert und blasenfrei gemacht.

5,00 Teile der erhaltenen Paste werden mit 0,51 Teilen Härterpaste auf einem Anmischblock in üblicher Weise homogen verrieben, wobei sich die Pasten nur widerstrebend mit dem Spatel vermischen lassen und zum Fadenziehen neigen. Die Zeichnungsschärfe an oralen Flächen, besonders bei Übergängen von Zahnhälsen zur Schleimhaut und sogenannten Taschen, ist gering.

Das vulkanisierende Gemisch unmittelbar nach dem Vermischen läßt sich nur mit großen Mühen aus einer Applikationsspritze ausbringen.

### Beispiel 4

800 Teile des in DE-OS 44 39 769 beschriebenen Harzes werden mit 65 Teilen eines Endgruppen essigsäureveresterten Polytetrahydrofurans mit einem Molgewicht von 4.500 und 5 Teilen Cabosil TS 610 in einem Behälter bei 80°C intensiv homogenisiert.

Anschließend wird die heiße Vormischung analog dem erfindungsgemäßen Beispiel 1 schockgekühlt.

Die Vormischung wird sodann mit 77 Teilen Kieselgur Celatom zu einer Paste verknetet, anschließend homogenisiert und blasenfrei gemacht.

5,00 Teile der erhaltenen Paste werden mit 0,51 Teilen Härterpaste auf einem Anmischblock in üblicher Weise homogen verrieben, wobei sich die Pasten leicht mit dem Spatel vermischen lassen und nicht fadenziehend sind. Die Zeichnungsschärfe an oralen Flächen, besonders bei Übergängen von Zahnhälsen zur Schleimhaut und sogenannten Taschen, ist sehr hoch.

Das vulkanisierende Gemisch unmittelbar nach dem Vermischen läßt sich sehr gut aus einer Applikationsspritze ausbringen.

### Standfestigkeitsbestimmung

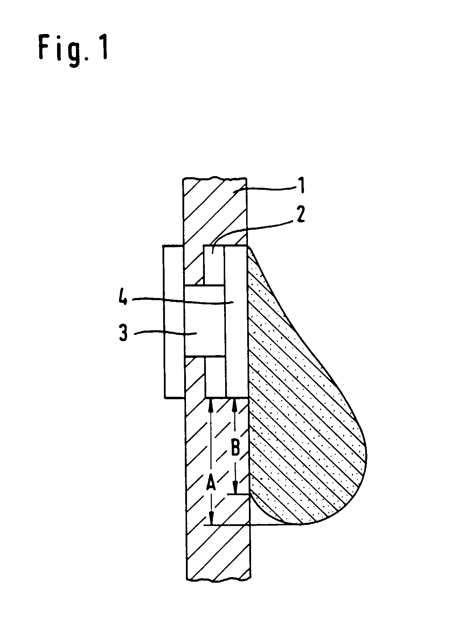

Zur Bestimmung der Standfestigkeit der angemischten Massen gleicher Abbindezeit wurde die in Fig. 1 schematisch dargestellte Prüfvorrichtung verwendet. Die Prüfvorrichtung weist eine Metallplatte 1 mit einer zylindrischen Vertiefung 2 auf. In der Vertiefung 2 ist ein Stempel 3 mit einer Stempelplatte 4 hin und her schiebbar. In der in Fig. 1 dargestellten ausgefahrenen Stellung schließt die Stempelplatte 4 bündig mit der in Fig. 1 rechts liegenden Oberfläche der Metallplatte 1 ab. Durch Verschieben des Stempels 3 nach links in Fig. 1 entsteht eine zylindrische Vertiefung in der Metallplatte 1.

Zum Beschicken der Prüfvorrichtung wird diese so angeordnet, daß die Vertiefung 2 nach oben zeigt und der Stempel 3 in diese Vertiefung zurückgezogen ist. Der von der Stempelplatte 4 freigegebene Teil der Vertiefung 2 wird mit einer im vorgesehenen Verhältnis angemischten Masse aus Basis- und Katalysatorpaste gefüllt und glatt gestrichen. Etwa 20-60 Sekunden nach Mischbeginn (in den vorliegenden Beispielen 60 Sekunden nach Mischbeginn) wird die Prüfvorrichtung in die Fig. 1 gezeigte senkrechte Stellung gebracht und der Stempel 3 herausgedrückt. Anschließend wartet man, bis die Mischung ausgehärtet ist.

Das Fließverhalten bzw. die Standfestigkeit der Mischung ergibt sich aus der Strecke A (die gesamte Fließstrecke der Mischung während der Aushärtzeit) und B (Fließstrecke während der Aushärtzeit, über die die Silikonmasse noch mit der Prüfwand in Kontakt bleibt). Je geringer die Differenz zwischen A und B ist und je kleiner die absoluten Werte von A und B sind, desto größer ist die Standfestigkeit des Materials.

Fig. 2 zeigt das so gemessene Fließverhalten einer gemäß Beispiel 2 bzw. Vergleichsbeispiel 2 hergestellten kondensationsvernetzenden Silikonmasse. Die Standfestigkeit der erfindungsgemäßen Masse (A) beträgt 1 mm (Strecke A = 4 mm, Strecke B = 3 mm). Dem gegenüber beträgt die Standfestigkeit des Vergleichsbeispiels 2 nach diesem Verfahren 2 mm (Strecke A = 14 mm, Strecke B = 12 mm). Ähnliche Meßwerte ergeben sich auch für die Massen gemäß Beispiel 1 bzw. Vergleichsbeispiel 1 (in den Fig. nicht dargestellt).

## Patentansprüche

1. Bei Raumtemperatur vulkanisierbare Masse auf der Basis von Polysiloxanen, Polysulfiden und/oder aziridinfreien Polyethern, gekennzeichnet durch einen Gehalt von wenigstens einem Wachs mit Ausnahme von hydriertem Rizinusöl, Paraffin- und Mikrowachsen, erhältlich durch Erhitzen einer Mischung der Polysiloxane, Polysulfide und/oder aziridinfreien Polyethermaterialien sowie des Wachses auf eine Temperatur oberhalb des Schmelzpunktes des Wachses, Herstellen einer Emulsion durch Dispergieren und anschließendes Schockkühlen der Emulsion.

2. Bei Raumtemperatur vulkanisierbare Masse auf der Basis von Polysiloxanen, Polysulfiden und/oder aziridinfreien Polyethern, gekennzeichnet durch einen Gehalt von wenigstens einem Wachs mit Ausnahme von hydriertem Rizinusöl, Paraffin- und Mikrowachsen, wobei das Wachs in der Masse ein kristallines Netzwerk bildet.

3. Masse nach Anspruch 1 oder 2, dadurch gekennzeichnet, daß der Wachsgehalt 1-40 Gew.-%, vorzugsweise 2-15 Gew.-%, weiter vorzugsweise 5-10 Gew.-% beträgt.

4. Masse nach einem der Ansprüche 1 bis 3, dadurch gekennzeichnet, daß das Molekulargewicht der Wachse kleiner als 2000 ist.

5. Masse nach einem der Ansprüche 1 bis 4, dadurch gekennzeichnet, daß die Wachse Estergruppen enthalten.

6. Masse nach einem der Ansprüche 1 bis 5, dadurch gekennzeichnet, daß sie ein additionsvernetzendes Polysiloxan enthält.

7. Masse nach einem der Ansprüche 1 bis 5, dadurch gekennzeichnet, daß sie ein kondensationsvernetzendes Polysiloxan enthält.

8. Verwendung einer Masse nach einem der Ansprüche 1 bis 7 als Abform-, Dublier- oder Modelliermasse.

9. Kit zur Herstellung einer Masse nach einem der Ansprüche 1 bis 6, dadurch gekennzeichnet, daß es zwei Komponenten enthält, die jeweils folgende Bestandteile aufweisen:
Komponente 1:
- Polysiloxane mit mindestens zwei ungesättigten Kohlenwasserstoffgruppen, vorzugsweise Vinylgruppen, im Molekül,
- Hydrogenpolysiloxane mit mindestens zwei Si-H-Gruppen im Molekül,
- Wachse mit Ausnahme von hydriertem Rizinusöl, Paraffin- oder Mikrowachsen,
- gegebenenfalls übliche Füllstoffe sowie Zusatz-, Hilfs- und Farbstoffe,
Komponente 2:
- gegebenenfalls Polysiloxane mit mindestens zwei ungesättigten Kohlenwasserstoffgruppen, vorzugsweise Vinylgruppen, im Molekül,
- Katalysator,
- gegebenenfalls Wachse mit Ausnahme hydriertem Rizinusöl, von Paraffin- oder Mikrowachsen,
- gegebenenfalls übliche Füllstoffe sowie Zusatz-, Hilfs- und Farbstoffe,
wobei die Wachse in jeder wachshaltigen Komponente ein kristallines Netzwerk bilden.

10. Kit nach Anspruch 9, dadurch gekennzeichnet, daß der Wachsgehalt in jeder der beiden Komponenten 1-40 Gew.-%, vorzugsweise 2-15 Gew.-%, weiter vorzugsweise 5-10 Gew.-% beträgt.

11. Kit zur Herstellung einer Masse nach einem der Ansprüche 1 bis 5 oder 7, dadurch gekennzeichnet, daß es zwei Komponenten enthält, die jeweils folgende Bestandteile aufweisen:
Komponente 1:
- Polysiloxane mit mindestens zwei Hydroxygruppen im Molekül,
- Wachse mit Ausnahme von Paraffin- oder Mikrowachsen,
- gegebenenfalls übliche Füllstoffe sowie Zusatz-, Hilfs- und Farbstoffe,
Komponente 2:
- Polyalkoxysilicate mit mindestens zwei Alkoxygruppen im Molekül,
- Katalysator,
- gegebenenfalls Wachse mit Ausnahme von Paraffin- oder Mikrowachsen,
- gegebenenfalls übliche Füllstoffe sowie Zusatz-, Hilfs- und Farbstoffe.
wobei die Wachse in jeder wachshaltigen Komponente ein kristallines Netzwerk bilden.

12. Kit nach Anspruch 11, dadurch gekennzeichnet, daß der Wachsgehalt in der nach Vermischen der Komponenten 1 und 2 im vorgesehenen Verhältnis erhaltenen Masse 1-40 Gew.-%, vorzugsweise 2-15 Gew.-%, weiter vorzugsweise 5-10 Gew.-% beträgt.

13. Kit nach einem der Ansprüche 9 bis 12, dadurch gekennzeichnet, daß die verwendeten Polysiloxane Polydimethylsiloxane sind.

14. Verfahren zur Herstellung der wachshaltigen Komponenten eines zur Herstellung einer aziridinfreien, bei Raumtemperatur vulkanisierbaren Masse vorgesehenen Kits, gekennzeichnet durch folgende Schritte:
- Erhitzen einer Mischung von Polysiloxanen, Polysulfiden und/oder aziridinfreien Polyethermaterialien sowie von Wachs, mit Ausnahme von Paraffin- oder Mikrowachsen, sowie gegebenenfalls Füll-, Zusatz-, Hilfs- und Farbstoffen auf eine Temperatur oberhalb des Schmelzpunktes des Wachses oder der Wachse,
- Herstellen einer Emulsion von Wachs, mit Ausnahme von Paraffin- oder Mikrowachsen, und Polysiloxanen, Polysulfiden und/oder aziridinfreien Polyethern durch Dispergieren,
- Schockkühlen der Emulsion,
- gegebenenfalls Einmischen weiterer Füll-, Zusatz-, Hilfs- und Farbstoffe in die abgekühlte Masse.

15. Verfahren nach Anspruch 14, dadurch gekennzeichnet, daß das Schockkühlen der Emulsion durch Aufbringen auf eine kalte Oberfläche, vorzugsweise durch Aufbringen auf eine Kühlwalze, erfolgt.

## Claims

1. A compound which can be vulcanised at room temperature, based on polysiloxanes, polysulphides and/or aziridine-free polyethers, characterised by a content of at least one wax, with the exception of hydrogenated castor oil, paraffin and microcrystalline waxes, obtainable by heating a mixture of the polysiloxanes, polysulphides and/or aziridine-free polyether materials, and also of the wax, to a temperature above the melting point of the wax, preparing an emulsion by dispersion, and subsequent rapid cooling of the emulsion.

2. A compound which can be vulcanised at room temperature, based on polysiloxanes, polysulphides and/or aziridine-free polyethers, characterised by a content of at least one wax, with the exception of hydrogenated castor oil, paraffin and microcrystalline waxes, wherein the wax forms a crystalline network in the compound.

3. A compound according to Claim 1 or 2, characterised in that the wax content is 1-40 % by weight, preferably 2-15 % by weight, more preferably 5-10 % by weight.

4. A compound according to any one of Claims 1 to 3, characterised in that the molecular weight of the wax is lower than 2000.

5. A compound according to any one of Claims 1 to 4, characterised in that the waxes contain ester groups.

6. A compound according to any one of Claims 1 to 5, characterised in that it contains an addition-cross linking polysiloxane.

7. A compound according to any one of Claims 1 to 5, characterised in that it contains a condensation-cross linking polysiloxane.

8. Use of a compound according to any one of Claims 1 to 7, as a moulding, doubling or modelling compound.

9. A kit for preparing a compound according to any one of Claims 1 to 6, characterised in that it contains two components which each have the following constituents:
component 1:
- polysiloxanes with at least two unsaturated hydrocarbon groups, preferably vinyl groups, in the molecule,
- hydrogen polysiloxanes with at least two Si-H groups in the molecule,
- waxes, with the exception of hydrogenated castor oil, paraffin and microcrystalline waxes,
- optionally conventional fillers, and also additives, auxiliary agents and colourants,
component 2:
- optionally, polysiloxanes with at least two unsaturated hydrocarbon groups, preferably vinyl groups, in the molecule,
- catalyst,
- optionally, waxes, with the exception of hydrogenated castor oil, paraffin or microcrystalline waxes,
- optionally conventional fillers, and also additives, auxiliary agents and colourants,
wherein the waxes form a crystalline network in each wax-containing component.

10. A kit according to Claim 9, characterised in that the wax content in each of the two components is 1-40 % by weight, preferably 2-15 % by weight, more preferably 5-10 % by weight.

11. A kit for preparing a compound according to any one of Claims 1 to 5 or 7, characterised in that it contains two components which each have the following constituents:
component 1:
- polysiloxanes with at least two hydroxy groups in the molecule,
- waxes, with the exception of paraffin or microcrystalline waxes,
- optionally conventional fillers, and also additives, auxiliary agents and colourants,
component 2:
- polyalkoxy silicates with at least two alkoxy groups in the molecule,
- catalyst,
- optionally, waxes, with the exception of paraffin or microcrystalline waxes,
- optionally conventional fillers, and also additives, auxiliary agents and colourants,
wherein the waxes form a crystalline network in each wax-containing component.

12. A kit according to Claim 11, characterised in that the wax content in the compound obtained after mixing the components 1 and 2 in the proportion specified is 1-40 % by weight, preferably 2-15 % by weight, more preferably 5-10 % by weight.

13. A kit according to any one of Claims 9 to 12, characterised in that the polysiloxanes used are polydimethyl siloxanes.

14. A method of preparing the wax-containing components of a kit intended for the preparation of an aziridine-free compound which can be vulcanised at room temperature, characterised by the following steps:
- heating a mixture of polysiloxanes, polysulphides and/or aziridine-free polyether materials, and also of wax, with the exception of paraffin or microcrystalline waxes, and also, optionally, fillers, additives, auxiliary agents and colourants, to a temperature above the melting point of the wax or waxes,
- preparing an emulsion of wax, with the exception of paraffin or microcrystalline waxes, and polysiloxanes, polysulphides and/or aziridine-free polyethers by dispersion,
- rapid cooling of the emulsion,
- optionally, incorporating further fillers, additives, auxiliary agents and colourants into the cooled compound.

15. A method according to Claim 14, characterised in that the rapid cooling of the emulsion is carried out by application to a cold surface, preferably by application to a cooling roller.

## Revendications

1. Masse à base de polysiloxanes, de polysulfures et/ou de polyéthers exempts d'aziridine pouvant être réticulée à température ambiante, caractérisée par une teneur d'au moins une cire à l'exception d'huile de ricin hydrogénée, de cire de paraffine et de microcires, obtenue par l'échauffement d'un mélange des polysiloxanes, polysulfures et/ou polyéthers exempts d'aziridine ainsi que de la cire à une température supérieure au point de fusion de la cire, par la réalisation d'une émulsion par dispersion puis par le refroidissement ultrarapide de l'émulsion.

2. Masse à base de polysiloxanes, de polysulfures et/ou de polyéthers exempts d'aziridine pouvant être réticulée à température ambiante, caractérisée par une teneur d'au moins une cire à l'exception d'huile de ricin hydrogénée, de cire de paraffine et de microcires, où la cire constitue dans la masse une structure réticulée cristalline.

3. Masse selon la revendication 1 ou 2, caractérisée en ce que la teneur en cire s'élève à 1-40 % en poids, de préférence à 2-15 % en poids, et encore de préférence à 5-10 % en poids.

4. Masse selon l'une des revendications 1 à 3, caractérisée en ce que le poids moléculaire des cires est inférieur à 2000.

5. Masse selon l'une des revendications 1 à 4, caractérisée en ce que les cires contiennent des groupes d'ester.

6. Masse selon l'une des revendications 1 à 5, caractérisée en ce qu'elle contient un polysiloxane réticulé par addition.

7. Masse selon l'une des revendications 1 à 5, caractérisée en ce qu'elle contient un polysiloxane réticulé par condensation.

8. Mise en oeuvre d'une masse selon l'une des revendications 1 à 7 sous forme de masse de moulage, de doublage ou de modelage.

9. Kit pour la réalisation d'une masse selon l'une des revendications 1 à 6, caractérisé en ce qu'il comprend deux composants présentant respectivement les éléments constitutifs suivants :
Composant 1 :
- des polysiloxanes avec au moins deux groupes d'hydrocarbures non saturés, de préférence des groupes de vinyle, dans la molécule ;
- des polysiloxanes d'hydrogène avec au moins deux groupes de Si-H dans la molécule ;
- des cires à l'exception de l'huile de ricin hydrogénée, de la cire de paraffine ou des microcires ;
- le cas échéant, des matières de remplissage habituelles ainsi que des matières additionnelles, auxiliaires et colorantes.
Composant 2 :
- le cas échéant, des polysiloxanes avec au moins deux groupes d'hydrocarbures non saturés, de préférence des groupes de vinyle, dans la molécule ;
- un catalyseur ;
- le cas échéant, des cires à l'exemption de l'huile de ricin hydrogénée, de la cire de paraffine ou des microcires ;
- le cas échéant, des matières de remplissage habituelles ainsi que des matières additionnelles, auxiliaires et colorantes ;
les cires constituant une structure réticulée cristalline dans chacun des composants cireux.

10. Kit selon la revendication 9, caractérisé en ce que la teneur en cire dans chacun des deux composants s'élève à 1-40 % en poids, de préférence à 2-15 % en poids, et encore de préférence à 5-10 % en poids.

11. Kit pour la réalisation d'une masse selon l'une des revendications 1 à 5 ou 7, caractérisé en ce qu'il comprend deux composants présentant respectivement les éléments constitutifs suivants :
Composant 1 :
- des polysiloxanes avec au moins deux groupes d'hydroxydes dans la molécule ;
- des cires à l'exception de la cire de paraffine ou de microcires ;
- le cas échéant, des matières de remplissage habituelles ainsi que des matières additionnelles, auxiliaires et colorantes ;
Composant 2 :
- des silicates polyalkoxydes avec au moins deux groupes d'alkoxydes dans la molécule ;
- un catalyseur ;
- le cas échéant, des cires à l'exception de la cire de paraffine ou des microcires ;
- le cas échéant, des matières de remplissage habituelles ainsi que des matières additionnelles, auxiliaires et colorantes ;
les cires constituant une structure réticulée cristalline dans chacun des composants cireux.

12. Kit selon la revendication 11, caractérisé en ce que la teneur en cire dans la masse obtenue par mélange des composants 1 et 2 dans les proportions prévues s'élève à 1-40 % en poids, de préférence à 2-15 % en poids, et encore de préférence à 5-10 % en poids.

13. Kit selon l'une des revendications 9 à 12, caractérisé en ce que les polysiloxanes utilisés sont des siloxanes polydiméthyliques.

14. Procédé de réalisation des composants cireux d'un kit prévu pour la réalisation d'une masse sans aziridine pouvant être réticulée à température ambiante, caractérisé par les étapes suivantes :
- échauffement d'un mélange de polysiloxanes, de polysulfures et/ou de polyéthers exempts d'aziridine ainsi que de cire, à l'exception de la cire de paraffine ou des microcires, ainsi que, le cas échéant, de matières de remplissage, additionnelles, auxiliaires et colorantes, à une température supérieure au point de fusion de la cire ou des cires ;
- réalisation par dispersion d'une émulsion de cire, à l'exception de cire de paraffine ou de microcires, de polysiloxanes, de polysulfures et/ou de polyéthers exempts d'aziridine ;
- refroidissement ultrarapide de l'émulsion ;
- le cas échéant, introduction dans la masse refroidie d'autres matières de remplissage, additionnelles, auxiliaires et colorantes.

15. Procédé selon la revendication 14, caractérisé en ce que le refroidissement ultrarapide de l'émulsion est réalisé par application sur une surface froide, de préférence par application sur un cylindre de refroidissement.
